# EUROPEAN PATENT SPECIFICATION

(11) **EP 4 122 911 B1**
(45) Date of publication and mention of the grant of the patent: **05.06.2024**
(21) Application number: 21900814.1
(22) Date of filing: 27.10.2021
(51) Int. Cl.: C07C 51/44, C07C 57/04, C07C 45/78, C07C 51/42, C07C 47/06, C07C 51/377

(54) **PROCESS FOR PRODUCING ACRYLIC ACID**
VERFAHREN ZUR HERSTELLUNG VON ACRYLSÄURE
PROCÉDÉ DE PRODUCTION D'ACIDE ACRYLIQUE

(30) Priority: 03.12.2020 KR 20200167641
(43) Date of publication of application: 25.01.2023
(73) Proprietor: LG Chem, Ltd., Seoul 07336 (KR)
(72) Inventor: KIM, Hyebin, Daejeon 34122 (KR); KIM, Mi Kyung, Daejeon 34122 (KR); KIM, Eunkyo, Daejeon 34122 (KR); SHIN, Joon Ho, Daejeon 34122 (KR)
(74) Representative: Goddar, Heinz J.
(86) International application number: PCT/KR2021/015172
(87) International publication number: WO 2022/119126

(56) References cited:
- EP-B1- 2 456 743
- CN-B- 101 255 109
- CN-U- 211 420 023
- GB-A- 1 458 396
- JP-A- 2014 189 510
- JP-B2- 6 772 376
- KR-A- 20060 048 785
- KR-A- 20110 113 036
- KR-A- 20170 113 177
- US-A1- 2016 107 974

## Description

### [Technical Field]

This application claims priority to and the benefits of Korean Patent Application No. 10-2020-0167641, filed with the Korean Intellectual Property Office on December 3, 2020.

The present application relates to a process for producing acrylic acid.

### [Background Art]

Acrylic acid has been generally produced through an oxidative dehydrogenation reaction of propylene, and demands on acrylic acid have increased as a raw material of super absorbent polymers, paints, adhesives and the like. Particularly, super absorbent polymers are used as hygiene products such as diapers.

So far, a considerable number of chemical products have been produced using raw materials derived from fossil raw materials such as coal or petroleum. However, using recyclable bio-derived resources as a carbon source has recently received attention as a substitute for existing fossil raw materials in terms of preventing global warming and protecting the environment. For example, development of methods using biomass resources including starch-based biomass such as corn or wheat, carbohydrate-based biomass such as sugar cane, cellulose-based biomass such as residue of rapeseed or rice straw, and the like as a raw material has been attempted.

In order words, studies on breaking from existing petrochemical-based manufacturing processes and producing chemical products based on environmental-friendly raw materials to obtain excellent properties in terms of environmental protection while obtaining sustainability are recently in progress.

One type of reactions producing other chemical products from lactic acid may include a gas-phase reaction in which a raw material including lactic acid is evaporated and brought into contact with a catalyst in a gaseous state to obtain a product. For example, as a technology of producing acrylic acid using lactic acid, a gas-phase dehydration reaction using a solid catalyst is known, and the dehydration reaction of lactic acid is mainly studied as a gas-phase reaction.

Lactic acid is a substance that polymerizes as an esterification reaction occurs in a liquid phase without a catalyst in the absence of water, and reacts as a lactic acid oligomer as lactic acid is concentrated and a concentration thereof increases. Dehydration occurs as lactic acid is oligomerized, and an oligomerization reaction of lactic acid occurs as the lactic acid is concentrated without water. When the lactic acid oligomer is introduced to a reactor for producing acrylic acid, fouling occurs in the reactor and the reaction yield decreases, and therefore, studies on a method to decrease the content of lactic acid oligomer for producing acrylic acid is in progress.

In addition to such a problem, economic feasibility needs to be enhanced in developing the process since reactions of bio-raw materials show low acrylic acid selectivity compared to existing petrochemical reactions such as an oxidation reaction of propylene.

Particularly, when producing bio-raw material-based acrylic acid, by-products such as carbon monoxide, carbon dioxide and acetaldehyde, which are by-products having a low boiling point, are produced together with acrylic acid production lowering acrylic acid selectivity, and accordingly, studies on a process to smoothly separate by-products such as low boiling point by-products and acrylic acid and increase purity of the low boiling point by-products themselves for commercialization when producing bio-raw material-based acrylic acid are in progress.

### Prior Art Documents

### Patent Documents

International Patent Application Laid-Open Publication No. 2005-095320

### [Disclosure]

### [Technical Problem]

GB1458396A discloses (page 2, column 2, line 102 - page 3, column 2, line 78) a process of purification of acrylic acid produced by oxidation of propene, and disclosing scrubbers and a distillation tower to separate acrylic acid and acrolein. This document mentions acetaldehyde as a secondary product.

The present application is directed to providing a process for producing acrylic acid.

### [Technical Solution]

One embodiment of the present application provides a process for producing acrylic acid, the process including a step 1 of separating a first low-boiling-point material including acetaldehyde (ACHO) and a first high-boiling-point material including acrylic acid (AA) by adding a first absorbent to a reaction product of a bio-raw material and cooling the result; a step 2 of separating a first incompressible material and a second low-boiling-point material including acetaldehyde (ACHO) by adding a second absorbent to the first low-boiling-point material including acetaldehyde (ACHO) and cooling the result; a step 3 of heating the second low-boiling-point material including acetaldehyde (ACHO); a step 4 of separating the heated second low-boiling-point material including acetaldehyde (ACHO) to acetaldehyde (ACHO) and the second absorbent; and a step 5 of producing acrylic acid by purifying the first high-boiling-point material including acrylic acid (AA).

### [Advantageous Effects]

A process for producing acrylic acid according to one embodiment of the present application includes a step 3 of heating a second low-boiling-point material including acetaldehyde (ACHO), which increases a temperature of the second low-boiling-point material discharged through a step 2 and introduces the result to a step 4, and energy efficiency of a separation tower increases when separating in the step 4.

Furthermore, in the process for producing acrylic acid according to the present application, a calorie of a heat source for heating the second low-boiling-point material including acetaldehyde (ACHO) uses a cooling calorie for cooling a second absorbent according to the present application, and a temperature of the second low-boiling-point material can be increased without a separate heating calorie.

### [Description of Drawings]

FIG. 1 is a schematic diagram illustrating a process for producing acrylic acid according to one embodiment of the present application.
FIG. 2 is a schematic diagram illustrating a step 2 to a step 4 of the process for producing acrylic acid according to one embodiment of the present application.
FIG. 3 is a schematic diagram illustrating a process for producing acrylic acid according to a comparative example of the present application.

### <Reference Numeral>

- 1:: Reaction Product of Bio-Raw Material
- 2:: First Absorbent
- 3:: First High-Boiling-Point Material
- 4:: First Low-Boiling-Point Material
- 5:: Second Absorbent
- 6:: Second Absorbent (Circulation Flow)
- 7:: First Incompressible Material
- 8:: Second Low-Boiling-Point Material
- 8-1:: Heated Second Low-Boiling-Point Material
- 9:: Acetaldehyde
- A:: Cooling Tower
- B:: Absorption Tower
- C:: Separation Tower
- D:: Heat Exchanger

### [Mode for Disclosure]

Hereinafter, the present specification will be described in more detail.

In the present specification, a description of a certain part "including" certain constituents means capable of further including other constituents, and does not exclude other constituents unless particularly stated on the contrary.

In the present specification, 'p to q' means a range of `greater than or equal to p and less than or equal to q'.

Hereinafter, embodiments of the present disclosure will be described in detail with reference to accompanying drawings so that those having common knowledge in the art may readily implement the present disclosure.

One embodiment of the present application provides a process for producing acrylic acid, the process including a step 1 of separating a first low-boiling-point material including acetaldehyde (ACHO) and a first high-boiling-point material including acrylic acid (AA) by adding a first absorbent to a reaction product of a bio-raw material and cooling the result; a step 2 of separating a first incompressible material and a second low-boiling-point material including acetaldehyde (ACHO) by adding a second absorbent to the first low-boiling-point material including acetaldehyde (ACHO) and cooling the result; a step 3 of heating the second low-boiling-point material including acetaldehyde (ACHO); a step 4 of separating the heated second low-boiling-point material including acetaldehyde (ACHO) to acetaldehyde (ACHO) and the second absorbent; and a step 5 of producing acrylic acid by purifying the first high-boiling-point material including acrylic acid (AA).

The process for producing acrylic acid according to one embodiment of the present application includes the step 3 of heating the second low-boiling-point material including acetaldehyde (ACHO), which increases a temperature of the second low-boiling-point material discharged through the step 2 and introduces the result to the step 4, and energy efficiency of a separation tower increases when separating in the step 4.

Furthermore, in the process for producing acrylic acid according to the present application, a calorie of a heat source for heating the second low-boiling-point material including acetaldehyde (ACHO) uses a cooling calorie for cooling the second absorbent according to the present application, and a temperature of the second low-boiling-point material may be increased without a separate heating calorie.

One embodiment of the present application provides the step 1 of separating a first low-boiling-point material including acetaldehyde (ACHO) and a first high-boiling-point material including acrylic acid (AA) by adding a first absorbent to a reaction product of a bio-raw material and cooling the result.

The reaction of the bio-raw material included in the step 1 may include a dehydration reaction of lactic acid, and may include any reaction without limit as long as it is a reaction of a bio-raw material for producing acrylic acid.

In one embodiment of the present application, the bio-raw material may be lactic acid in a gas phase.

In one embodiment of the present application, the gas phase may mean a vaporized state, that is, a state in which a liquid is vaporized to become a gas.

In the present application, the lactic acid is an organic compound having an asymmetric carbon atom to which four atomic groups of a carboxyl group, a hydroxyl group, a methyl group and hydrogen bond, includes both D-lactic acid and L-lactic acid, and may mean a single lactic acid monomer.

In the present application, a lactic acid oligomer means a material obtained by lactic acid reacting to each other to form a dimer, a trimer and the like, and the lactic acid oligomer may mean a dimer to a 100-mer of lactic acid.

Lactic acid is a substance that polymerizes through an esterification reaction in a liquid phase without a catalyst even in the absence of water, and substances formed through a polymerization reaction of lactic acid may all be expressed as a lactic acid oligomer. In other words, all substances formed through a polymerization reaction of lactic acid other than a single lactic acid monomer may be defined as a lactic acid oligomer.

In the process for producing acrylic acid provided in one embodiment of the present application, the vapor phase lactic acid includes water; and a lactic acid raw material, and
the lactic acid raw material includes lactic acid; and a lactic acid oligomer, and the lactic acid raw material is included in greater than or equal to 10 parts by weight and less than or equal to 100 parts by weight based on 100 parts by weight of the vapor phase lactic acid.

In another embodiment, the lactic acid raw material may be included in greater than or equal to 10 parts by weight and less than or equal to 100 parts by weight, preferably greater than or equal to 30 parts by weight and less than or equal to 100 parts by weight, and more preferably greater than or equal to 60 parts by weight and less than or equal to 100 parts by weight based on 100 parts by weight of the vapor phase lactic acid.

The vapor phase lactic acid is an aqueous lactic acid solution in a final vaporized state before producing acrylic acid, and by the lactic acid raw material content satisfying the above-mentioned range in the vapor phase lactic acid, the introduced amount of the lactic acid raw material itself is suitable, and the water content is adjusted to a proper range, and as a result, excellent economic feasibility is obtained in the process for producing acrylic acid according to the present application.

In the process for producing acrylic acid provided in one embodiment of the present application, a ratio of the lactic acid:lactic acid oligomer in the vapor phase lactic acid may be from 100:0 to 80:20.

In another embodiment, a ratio of the lactic acid:lactic acid oligomer in the vapor phase lactic acid may satisfy a range of 100:0 to 80:20, preferably 100:0 to 90:10 and more preferably 100:0 to 95:5.

In other words, the process for producing acrylic acid according to the present disclosure breaks from existing petrochemical-based manufacturing processes and produces acrylic acid based on lactic acid, an environmental-friendly bio-raw material, and as a result, excellent properties are obtained in terms of environmental protection while obtaining sustainability. The vapor phase lactic acid corresponds to the bio-raw material of the step 1 according to the present application, and fouling occurring in the reactor may be reduced for the process for producing final acrylic acid and the reaction yield may increase.

In one embodiment of the present application, the reaction product of the bio-raw material may include acrylic acid, acetaldehyde, carbon monoxide, carbon dioxide, water, hydrogen, a lactic acid monomer, acetic acid, 2,3-pentadione (2,3-PD) and propionic acid (PA).

Particularly, acetaldehyde is not produced in a petrochemical-based propylene oxidation reaction since the reaction temperature is from 250°C to 270°C, however, acetaldehyde is produced as a by-product in the process for producing acrylic acid since a dehydration reaction of the vapor phase lactic acid in the reaction of the bio-raw material according to the present application occurs at a high temperature (330°C to 400°C), and commercializing acetaldehyde as well produced as a by-product herein is a main purpose of the present disclosure.

In the process for producing acrylic acid provided in one embodiment of the present application, the step 1 includes a step of separating through a cooling tower, and the cooling tower has a cooling temperature of higher than or equal to 10°C and lower than or equal to 150°C and an inner pressure of greater than or equal to 0.5 bar and less than or equal to 5.0 bar.

In another embodiment, the cooling tower may have an inner pressure of greater than or equal to 0.5 bar and less than or equal to 5.0 bar, preferably greater than or equal to 1.0 bar and less than or equal to 4.0 bar and more preferably greater than or equal to 2.0 bar and less than or equal to 3.5 bar, and may specifically satisfy an inner pressure of 3.0 bar.

In another embodiment, the cooling tower may have an inner temperature of 10°C or higher, preferably 20°C or higher and more preferably 40°C or higher, and may be 200°C or lower and preferably 150°C or lower.

By the inner temperature and the inner pressure of the cooling tower satisfying the above-mentioned ranges in the step 1 as above, a content of acrylic acid included in the first low-boiling-point material discharged to an upper portion of the cooling tower may be minimized, that is, all acrylic acid in the reaction product of the bio-raw material is discharged to a lower portion of the cooling tower in the first high-boiling-point material including acrylic acid (AA), and as a result, yield and purity of the acrylic acid may increase.

In other words, in the process for producing acrylic acid, the step 1 may be a step of separating the first high-boiling-point material including acrylic acid and other low-boiling point by-products through cooling.

In the process for producing acrylic acid provided in one embodiment of the present application, the first absorbent is included so that acrylic acid (AA) included in the first low-boiling-point material of the step 1 is included in 1 parts by weight or less with respect to 100 parts by weight of acrylic acid in the reaction product of the bio-raw material.

In the step 1 according to the present disclosure, acrylic acid in the reaction product of the bio-raw material may all be discharged to a lower portion of the cooling tower in the first high-boiling-point material including acrylic acid (AA) by adjusting, as well as adjusting the temperature and pressure ranges of the cooling tower as described above, the content of the first absorbent.

Specifically, in one embodiment of the present application, acrylic acid (AA) included in the first low-boiling-point material of the step 1 may be included in 1 parts by weight or less, preferably 0.5 parts by weight or less and more preferably 0.01 parts by weight or less, and may be 0 parts by weight or greater and preferably 0.005 parts by weight or greater with respect to 100 parts by weight of acrylic acid in the reaction product of the bio-raw material.

In other words, acrylic acid (AA) included in the first low-boiling-point material of the step 1 is an amount discarded and not obtained, and by adjusting the amount of the first absorbent as above, the weight of acrylic acid (AA) included in the first low-boiling-point material is adjusted, and an economically superior process for producing acrylic acid may be provided.

In the process for producing acrylic acid provided in one embodiment of the present application, when the cooling tower has a cooling temperature of higher than or equal to 10°C and lower than or equal to 50°C, the first absorbent is included in greater than or equal to 1 parts by weight and less than or equal to 20 parts by weight with respect to 100 parts by weight of the reaction product of the bio-raw material of the step 1.

In another embodiment, the first absorbent may be included in greater than or equal to 1 parts by weight and less than or equal to 20 parts by weight, preferably greater than or equal to 2 parts by weight and less than or equal to 15 parts by weight, and more preferably greater than or equal to 3 parts by weight and less than or equal to 10 parts by weight with respect to 100 parts by weight of the reaction product of the bio-raw material of the step 1.

In the process for producing acrylic acid provided in one embodiment of the present application, when the cooling tower has a cooling temperature of higher than or equal to 50°C and lower than or equal to 80°C, the first absorbent is included in greater than or equal to 35 parts by weight and less than or equal to 50 parts by weight with respect to 100 parts by weight of the reaction product of the bio-raw material of the step 1.

In another embodiment, the first absorbent may be included in greater than or equal to 35 parts by weight and less than or equal to 50 parts by weight, preferably greater than or equal to 37 parts by weight and less than or equal to 45 parts by weight, and more preferably greater than or equal to 40 parts by weight and less than or equal to 45 parts by weight with respect to 100 parts by weight of the reaction product of the bio-raw material of the step 1.

The process for producing acrylic acid of the present application adjusts the amount of heat of the cooling tower when conducting the step 1 as above and includes the first absorbent in the above-mentioned content range, and, by particularly including the first absorbent in the above-mentioned range, adjusts the first high-boiling-point material including acrylic acid, water and the like to be all discharged to a lower portion of the absorption tower, and accordingly, finally produced acrylic acid has increased yield and purity, and acetaldehyde produced as a by-product may also be produced in high purity.

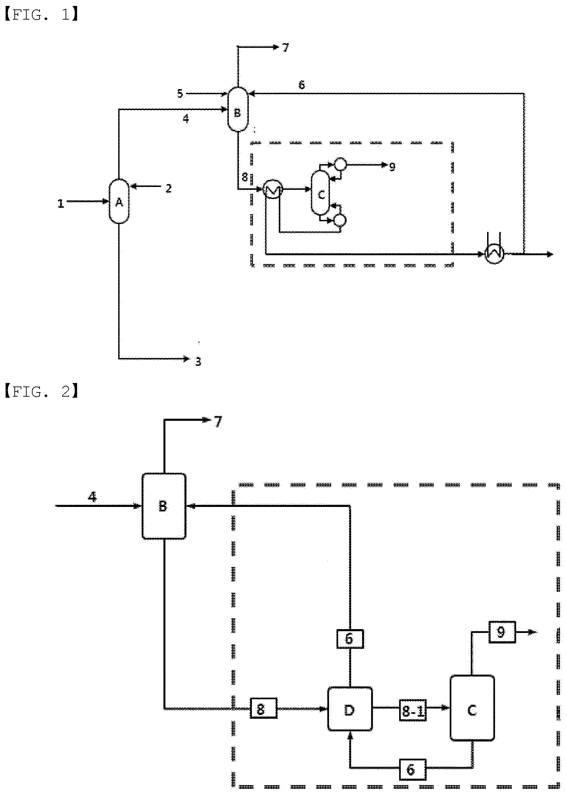

FIG. 1 is a schematic diagram of the process for producing acrylic acid according to the present application, and specifically, it is identified that the reaction product of the bio-raw material (1) is introduced to the cooling tower (A), and the boiling point-dependent separation process occurs by including the first absorbent (2), and it is identified herein that the first high-boiling-point material including acrylic acid (AA) (3) is separated to the lower portion and the first low-boiling-point material including acetaldehyde (ACHO) (4) is separated to the upper portion.

In the process for producing acrylic acid provided in one embodiment of the present application, the first absorbent includes a material having a boiling point difference of 20°C or higher compared to a normal boiling point (NBP) of the acrylic acid (AA) and a boiling point difference of 50°C or higher compared to a normal boiling point (NBP) of the acetaldehyde (ACHO).

In the present application, the normal boiling point (NBP) is a synonym for a boiling point, and may mean a boiling point of a liquid when an external pressure is 1 atmosphere (760 mmHg). A boiling point of a material normally means a normal boiling point, and for example, a normal boiling point of water may be expressed as 100°C. It means a temperature at which not only evaporation occurs from a liquid surface, but also vaporization occurs from inside a liquid and bubbles start to generate, and may mean a temperature at which a change occurs in a material state from a liquid to a gas.

In another embodiment, the first absorbent may be a material having a boiling point difference of higher than or equal to 20°C and lower than or equal to 40°C compared to a normal boiling point (NBP) of the acrylic acid (AA) and a boiling point difference of higher than or equal to 50°C and lower than or equal to 80°C compared to a normal boiling point (NBP) of the acetaldehyde (ACHO).

In one embodiment of the present application, the acrylic acid has a normal boiling point of 141°C, and the acetaldehyde has a normal boiling point of 20°C.

In one embodiment of the present application, the first absorbent may be a material having a boiling point difference of higher than or equal to 20°C and lower than or equal to 40°C compared to a normal boiling point (NBP) of the acrylic acid (AA), having a boiling point difference of higher than or equal to 50°C and lower than or equal to 80°C compared to a normal boiling point (NBP) of the acetaldehyde (ACHO), and having a higher boiling point compared to the acetaldehyde.

In one embodiment of the present application, the first absorbent may be used without limit as long as the above-mentioned conditions are satisfied, and specifically, the first absorbent may include water in one embodiment of the present application.

In one embodiment of the present application, the first absorbent may satisfy a temperature range of higher than or equal to 10°C and lower than or equal to 100°C.

In another embodiment, the first absorbent may satisfy a temperature range of higher than or equal to 10°C and lower than or equal to 100°C, preferably higher than or equal to 20°C and lower than or equal to 100°C, and most preferably higher than or equal to 30°C and lower than or equal to 50°C.

By the first absorbent temperature range satisfying the above-mentioned range as above, the first absorbent temperature is adjusted to a similar range as the range of the inner temperature of the cooling tower when included in the cooling tower of the step 1, which enhances economic feasibility by reducing inner capacity of the cooling tower.

In one embodiment of the present application, the acrylic acid included in the first high-boiling-point material may be included in 95 parts by weight or greater based on 100 parts by weight of acrylic acid included in the reaction product of the bio-raw material.

In another embodiment, the acrylic acid included in the first high-boiling-point material may be included in 95 parts by weight or greater, preferably 97 parts by weight or greater and more preferably 99 parts by weight or greater, and may be 100 parts by weight or less based on 100 parts by weight of acrylic acid included in the reaction product of the bio-raw material.

In one embodiment of the present application, the acetaldehyde included in the first low-boiling-point material may be included in 90 parts by weight or greater based on 100 parts by weight of acetaldehyde included in the reaction product of the bio-raw material.

In another embodiment, the acetaldehyde included in the first low-boiling-point material may be included in 90 parts by weight or greater, preferably 93 parts by weight or greater and more preferably 95 parts by weight or greater, and may be 100 parts by weight or less based on 100 parts by weight of acetaldehyde included in the reaction product of the bio-raw material.

One embodiment of the present application provides a step 2-1 of separating a second-1 low-boiling-point material including acetaldehyde (ACHO) and a second-1 high-boiling-point material including acrylic acid (AA) by distilling the first high-boiling-point material including acrylic acid (AA).

In one embodiment of the present application, the step 2-1 is a step of once more distilling the first high-boiling-point material including acrylic acid (AA) discharged to a lower portion of the cooling tower in the step 1, and corresponds to a step of separating a second-1 low-boiling-point material including acetaldehyde (ACHO) and a second-1 high-boiling-point material including acrylic acid (AA).

In other words, through such a process of the step 2-1, acetaldehyde that may be discharged to a lower portion of the cooling tower in the step 1 may be further separated to obtain high yield and high purity acrylic acid, and by obtaining a second-1 low-boiling-point material including acetaldehyde (ACHO), high yield and high purity acetaldehyde may also be obtained through a separation process of the step to describe later.

In one embodiment of the present application, the first high-boiling-point material including acrylic acid (AA) may include water; acrylic acid; and acetaldehyde.

In one embodiment of the present application, acrylic acid included in the second-1 high-boiling-point material may be included in 95 parts by weight or greater based on 100 parts by weight of the acrylic acid included in the first high-boiling-point material including acrylic acid (AA).

In another embodiment, acrylic acid included in the second-1 high-boiling-point material may be included in 95 parts by weight or greater, preferably 97 parts by weight or greater and more preferably 99 parts by weight or greater, and may be 100 parts by weight or less and preferably 99.99 parts by weight or less based on the 100 parts by weight of the acrylic acid included in the first high-boiling-point material including acrylic acid (AA).

In the process for producing acrylic acid according to the present disclosure, the yield of finally produced acrylic acid included in the second-1 high-boiling-point material may be high by going through the process of adding the absorbent and cooling in the step 1 and separating aldehyde once again in the step 2-1.

In one embodiment of the present application, the second-1 high-boiling-point material may be purified to obtain final acrylic acid.

In one embodiment of the present application, the acetaldehyde included in the second-1 low-boiling-point material may be included in 95 parts by weight or greater based on 100 parts by weight of the acetaldehyde included in the first high-boiling-point material including acrylic acid (AA).

In another embodiment, the acetaldehyde included in the second-1 low-boiling-point material may be included in 95 parts by weight or greater, preferably 96 parts by weight or greater and more preferably 97 parts by weight or greater, and may be 100 parts by weight or less and preferably 99.99 parts by weight or less based on 100 parts by weight of the acetaldehyde included in the first high-boiling-point material including acrylic acid (AA).

As described above, while obtaining acrylic acid in a high yield, acetaldehyde may be also commercialized by separating the acetaldehyde included in the first high-boiling-point material including acrylic acid (AA) again and going through a process to describe later.

Herein, in the step 5 to describe later, the first high-boiling-point material including acrylic acid (AA) or the second-1 low-boiling-point material including acrylic acid (AA) may be purified to obtain final acrylic acid.

One embodiment of the present application provides the step 2 of separating a first incompressible material and the second low-boiling-point material including acetaldehyde (ACHO) by adding a second absorbent to the first low-boiling-point material including acetaldehyde (ACHO) and cooling the result.

In the process for producing acrylic acid of the present application, the step 2 is a process of adding a second absorbent to the first low-boiling-point material discharged to an upper portion of the cooling tower in the step 1 and cooling the result, and through such a process, acetaldehyde produced as a by-product in the process for producing acrylic acid may also be commercialized. In other words, it is a step of commercializing high purity acetaldehyde as well while obtaining high purity acrylic acid, which is considered as a characteristic of the disclosure of the present application, and may be a main characteristic of the present disclosure.

In the process for producing acrylic acid provided in one embodiment of the present application, the step 2 includes a step of separating through an absorption tower, and the absorption tower has a temperature of is higher than or equal to 0°C and lower than or equal to 100°C and an inner pressure of greater than or equal to 0.1 bar and less than or equal to 10.0 bar.

In another embodiment, the absorption tower of the step 2 may have an inner pressure of greater than or equal to 0.1 bar and less than or equal to 10.0 bar, preferably greater than or equal to 1.0 bar and less than or equal to 8.0 bar and more preferably greater than or equal to 1.5 bar and less than or equal to 5.0 bar, and may specifically satisfy an inner pressure of 2.5 bar.

In another embodiment, the absorption tower of the step 2 may have an inner temperature of 0°C or higher, preferably 5°C or higher and more preferably 10°C or higher, and may be 100°C or lower and preferably 80°C or lower.

By the inner temperature and the inner pressure of the absorption tower satisfying the above-mentioned ranges in the step 2 as above, the acetaldehyde included in the first low-boiling-point material discharged to an upper portion of the absorption tower may be commercialized in high yield and high purity, and by the process of separating from the first incompressible material included in the first low-boiling-point material progressing smoothly in particular, acetaldehyde may be obtained in high yield and high purity while obtaining acrylic acid.

In the process for producing acrylic acid provided in one embodiment of the present application, the second absorbent is included so that acetaldehyde (ACHO) included in the first incompressible material of the step 2 is included in 1 parts by weight or less with respect to 100 parts by weight of acetaldehyde in the reaction product of the bio-raw material.

In the step 2 according to the present disclosure, acetaldehyde in the reaction product of the bio-raw material may all be discharged to a lower portion of the absorption tower in the first low-boiling-point material including acetaldehyde (ACHO) by adjusting the content of the second absorbent.

Specifically, in one embodiment of the present application, acetaldehyde (ACHO) included in the first incompressible material of the step 2 may be included in 1 parts by weight or less, preferably 0.7 parts by weight or less and more preferably 0.5 parts by weight or less, and may be 0 parts by weight or greater and preferably 0.005 parts by weight or greater with respect to 100 parts by weight of acetaldehyde in the reaction product of the bio-raw material.

In other words, another characteristic of the process for producing acrylic acid according to the present application is commercializing acetaldehyde produced as a by-product, and by adjusting the amount of the second absorbent as above, loss of the acetaldehyde may be minimized.

The process for producing acrylic acid of the present application includes the second absorbent in the above-mentioned content range when conducting the step 2 as above, and, by particularly including the second absorbent in the above-mentioned range, adjusts only the second low-boiling-point material including acetaldehyde to be discharged to a lower portion of the absorption tower of the step 2 in the first low-boiling-point material including acetaldehyde, an incompressible material and the like, and finally produced acetaldehyde has increased yield and purity.

In the process for producing acrylic acid provided in one embodiment of the present application, the second absorbent includes, as a material having a higher boiling point compared to a normal boiling point (NBP) of the acetaldehyde (ACHO), a material having a boiling point difference of 20°C or higher.

In the process for producing acrylic acid provided in another embodiment, the second absorbent includes, as a material having a higher boiling point compared to a normal boiling point (NBP) of the acetaldehyde (ACHO), a material having a boiling point difference of higher than or equal to 20°C and lower than or equal to 100°C.

In the process for producing acrylic acid provided in another embodiment, the second absorbent includes, as a material having a higher boiling point compared to a normal boiling point (NBP) of the acetaldehyde (ACHO), a material having a boiling point difference of higher than or equal to 20°C and lower than or equal to 100°C, preferably having a boiling point difference of higher than or equal to 30°C and lower than or equal to 90°C, and more preferably having a boiling point difference of higher than or equal to 50°C and lower than or equal to 80°C.

In another embodiment, the second absorbent may be a material having a higher boiling point compared to a boiling point of the acetaldehyde.

In one embodiment of the present application, the second absorbent may include one or more selected from the group consisting of water and acrylic acid.

In one embodiment of the present application, the second absorbent may satisfy a temperature range of higher than or equal to -5°C and lower than or equal to 20°C.

In another embodiment, the second absorbent may satisfy a temperature range of higher than or equal to -5°C and lower than or equal to 20°C, preferably higher than or equal to 5°C and lower than or equal to 15°C, and most preferably higher than or equal to 5°C and lower than or equal to 10°C.

By the second absorbent temperature range satisfying the above-mentioned range as above, the second absorbent temperature is adjusted to a similar range as the range of the inner temperature of the absorption tower when included in the absorption tower of the step 3, which enhances economic feasibility by reducing inner capacity of the absorption tower.

In other words, the temperature of the second absorbent may mean a temperature when, after separated in the step 4, included in the step 2 by the circulation process.

In one embodiment of the present application, the acetaldehyde included in the second low-boiling-point material may be included in 95 parts by weight or greater based on 100 parts by weight of the acetaldehyde included in the first low-boiling-point material.

In another embodiment, the acetaldehyde included in the second low-boiling-point material may be included in 95 parts by weight or greater, preferably 96 parts by weight or greater and more preferably 97 parts by weight or greater, and may be 100 parts by weight or less and preferably 99.9 parts by weight or less based on 100 parts by weight of the acetaldehyde included in the first low-boiling-point material.

In one embodiment of the present application, the first incompressible material may include carbon monoxide, carbon dioxide and an inert gas.

The step 2 of the present application may be identified in FIG. 1, and specifically, the process of supplying the first low-boiling-point material (4) to the absorption tower (B), and then supplying the second absorbent (5 and/or 6) to separate the first incompressible material (7) including an inert gas and the second low-boiling-point material (8) may be identified.

One embodiment of the present application may include the step 3 of heating the second low-boiling-point material including acetaldehyde (ACHO).

In the process for producing acrylic acid provided in one embodiment of the present application, the step 3 includes a step of heating through a heat exchanger, and as a heat source for the heating, a cooling calorie of the second absorbent of the step 4 is used as the heat source.

The second low-boiling-point material including acetaldehyde (ACHO) gone through the step 2 is in a state of being cooled and separated, and the second low-boiling-point material has a temperature of approximately 30°C to 80°C. Herein, introducing the second low-boiling-point material directly to the step 4 to describe later has a problem of causing a high load on a reboiler of the separation tower used in the step 4 since the temperature of the second low-boiling-point material is low compared to the temperature of the separation tower.

Accordingly, in the process for producing acrylic acid according to the present application, the second low-boiling-point material including acetaldehyde (ACHO) is heated and then introduced to the separation tower of the step 4 to reduce a load on the separation tower.

Particularly, as a heat source for heating the second low-boiling-point material, a cooling calorie itself of the second absorbent of the step 4 is used as the heat source, and in the present disclosure, the second low-boiling-point material may be heated without supplying an additional heat source.

Specifically, the step 3 may be identified in FIG. 2. FIG. 2 is a schematic diagram illustrating the step 2 to the step 4 in the process for producing acrylic acid according to one embodiment of the present application, and it is identified that the second low-boiling-point material (8) separated through the absorption tower (B) of the step 2 is heated by being introduced to the heat exchanger (D), and then the heated second low-boiling-point material (8-1) is supplied to the separation tower (C).

Herein, it is identified that the second absorbent (6) is separated to a lower portion of the separation tower (C) and circulated back to the absorption tower (B) of the step 2 by a liquid circulation flow, and the calorie for cooling the second absorbent (6) heated in the separation tower is used to heat the second low-boiling-point material (8). In other words, the heated second absorbent (6) needs to be cooled to be included again in the absorption tower (B) of the step 2, and heating the second low-boiling-point material (8) while cooling the heated second absorbent itself is a main characteristic of the present disclosure.

In other words, in FIG. 2, the temperature of the second low-boiling-point material (8) introduced to the heat exchanger (D) is low, and through the second absorbent (6) having a high temperature coming through the separation tower (C), the second low-boiling-point material may be in a heated state (8-1), and the second absorbent (6) may also be included in a liquid circulation flow in a state of having a lowered temperature after going through the heat exchanger (D).

In one embodiment of the present application, the second absorbent gone through the heat exchanger (D) may satisfy a temperature range of higher than or equal to -5°C and lower than or equal to 20°C, and may be included in the step 2 through a liquid circulation flow.

In the process for producing acrylic acid provided in one embodiment of the present application, the heated second low-boiling-point material including acetaldehyde (ACHO) has a temperature of higher than or equal to 60°C and lower than or equal to 180°C.

The second low-boiling-point material including acetaldehyde (ACHO) gone through the step 2 is formed to have a low temperature, and herein, a problem of causing a high road on the separation tower occurs in the process of separating the absorbent of the step 4 to describe later.

The process for producing acrylic acid according to one embodiment of the present application includes the step 3 of heating the second low-boiling-point material including acetaldehyde (ACHO) in order to resolve such a problem, and including the second low-boiling-point material in the step 4 after heating may resolve the problem of causing a load on the separation tower, and the temperature of the second low-boiling-point material is increased without a separate heating calorie by using a cooling calorie for cooling the second absorbent according to the present application as a calorie of a heat source for heating the second low-boiling-point material including acetaldehyde (ACHO), which are main characteristics of the present disclosure.

In one embodiment of the present application, the heated second low-boiling-point material including acetaldehyde (ACHO) may have a temperature of higher than or equal to 60°C and lower than or equal to 180°C, preferably higher than or equal to 65°C and lower than or equal to 160°C, and more preferably higher than or equal to 90°C and lower than or equal to 150°C.

By including the second low-boiling-point material including acetaldehyde (ACHO) in the step 4 after satisfying the above-mentioned temperature range, a problem of causing a load on the separation tower may be resolved.

One embodiment of the present application provides the step 4 of separating the heated second low-boiling-point material including acetaldehyde (ACHO) to acetaldehyde (ACHO) and the second absorbent.

In one embodiment of the present application, by including the step 4 as above, acetaldehyde used as a final product and the second absorbent may be separated.

In the process for producing acrylic acid provided in one embodiment of the present application, the step 4 includes a step of separating through a separation tower, and the separation tower has a temperature of higher than or equal to 10°C and lower than or equal to 200°C and an inner pressure of greater than or equal to 0.3 bar and less than or equal to 10.0 bar.

In another embodiment, the separation tower of the step has an inner pressure of greater than or equal to 0.3 bar and less than or equal to 10.0 bar, preferably greater than or equal to 1.0 bar and less than or equal to 8.0 bar and more preferably greater than or equal to 2.0 bar and less than or equal to 5.0 bar, and may specifically satisfy an inner pressure of 3.0 bar.

In another embodiment, the separation tower of the step has an inner temperature of 10°C or higher, preferably 20°C or higher and more preferably 40°C or higher, and may be 200°C or lower and preferably 150°C or lower.

By the inner temperature and the inner pressure of the separation tower satisfying the above-mentioned ranges as above, acetaldehyde included in the second low-boiling-point material discharged to a lower portion of the absorption tower of the step 2 and heated through the step 3 may be commercialized in high yield and high purity, and by particularly introducing the second low-boiling-point material after heating, a calorie of the separation tower that separates from the second absorbent may be minimized, and the process of separating from the second absorbent may progress smoothly, and as a result, acetaldehyde may be obtained in high yield and high purity while obtaining acrylic acid.

In addition, after separating acetaldehyde and the second absorbent as above, the second absorbent may be included again in the step 2 through a liquid flow, and the amount of the second absorbent used may also be minimized.

Herein, the process for producing acrylic acid according to one embodiment of the present application uses a cooling calorie for cooling the second absorbent as a heat source for heating of the step 3 as described above, and an efficient and economical process is provided without an additional heat supply.

By the second absorbent temperature satisfying the above-mentioned range as above, an adjustment made to absorb 99 wt% or greater of the acetaldehyde in the second low-boiling-point material including acetaldehyde (ACHO).

In the process for producing acrylic acid provided in one embodiment of the present application, purity of final acetaldehyde produced through the separation tower is 95% or greater.

In another embodiment, purity of the acetaldehyde may be 100% or less, and 99.99% or less.

The production process of the present disclosure is particularly useful for synthesizing acrylic acid, and specifically, the vapor composition including lactic acid obtained in the present disclosure may be brought into contact with a dehydration catalyst to prepare acrylic acid. A produced reaction gas is collected and liquefied by cooling or brining into contact with a collection liquid, and after going through a purification process such as extraction, distillation and crystallization, high purity acrylic acid may be obtained. Produced acrylic acid is widely used as a raw material of absorbent polymers, paints, adhesives or the like.

Hereinafter, examples of the present disclosure will be described in detail so that those having common knowledge in the art may readily implement the present disclosure. However, the present disclosure may be embodied in various different forms, and is not limited to the examples described herein.

### <Preparation Example>

The following examples and comparative examples were simulated by Aspen Plus of Aspen Technology Inc..

As a common process, a reaction product of a bio-raw material (acetaldehyde, acetic acid, water, inert gas, high-boiling-point material (2,3-PD, propionic acid or the like)) was introduced to a cooling tower of a step 1. Water (normal boiling point 100°C) was introduced therewith as a first absorbent. Herein, the cooling tower of the step 1 was operated at an inner temperature of approximately 40°C to 200°C and an inner pressure of 1.0 bar to 10.0 bar, and a first low-boiling-point material including acetaldehyde was separated to an upper portion of the cooling tower and a first high-boiling-point material including acrylic acid was separated to a lower portion of the cooling tower.

After that, the first low-boiling-point material including acetaldehyde was introduced to an absorption tower of a step 2, and water (normal boiling point 100°C) was introduced therewith as a second absorbent. In addition, a second absorbent by a liquid circulation flow was also included. The absorption tower of the step 2 was operated at a temperature of approximately 5°C to 100°C and an inner pressure of 1.0 bar to 10.0 bar. A first incompressible material was discharged to an upper portion of the absorption tower, and a second low-boiling-point material including acetaldehyde was separated to a lower portion of the absorption tower.

After that, processes of the following examples and comparative examples were conducted.

### <Example 1>

The first incompressible material (7) and the second low-boiling-point material (8) were separated in the absorption tower (B) as in FIG. 2, and herein, the second low-boiling-point material itself had a temperature of 45°C. After that, the second low-boiling-point material was introduced to a heat exchanger (D) and heated, and after the heating, the heated second low-boiling-point material (8-1) had a temperature of 115.4°C.

The heated second low-boiling-point material was used as a feed for a separation tower (C), and with a total feed flow rate of 20 ton/hr, acetaldehyde was included in 9.1 wt%, the second absorbent (water) in 90.8 wt% and CO₂ in 0.1 wt% or less. Herein, the separation tower was operated at a pressure of 4.0 bar and a calorie (Q) of 1.18 Gcal/hr.

The second absorbent (water) and acetaldehyde (9) were separated in the separation tower (C), and with a final acetaldehyde flow rate of 1.86 ton/hr, acetaldehyde was included in 97.7 wt%, the second absorbent (water) in 2.2 wt% and CO₂ in 0.1 wt% or less, and acetaldehyde was able to be commercialized as above.

The second absorbent (6) heated in the separation tower (C) was supplied back to the absorption tower (B) through a liquid flow, and herein, a calorie for cooling the heated second absorbent (6) was used as a heat source of the heat exchanger (D). Specifically, the second absorbent immediately after discharged from the separation tower (C) had a temperature of 143.7°C, and was cooled to 50.0°C as it is used as a heat source for heating the heat exchanger (D), and after that, was further cooled through cooling water and chilled water.

### <Comparative Example 1>

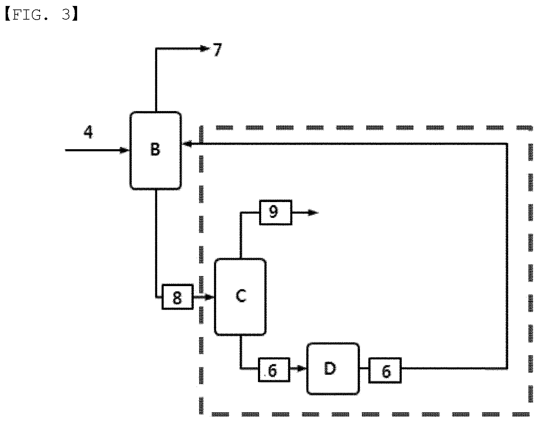

The first incompressible material (7) and the second low-boiling-point material (8) were separated in the absorption tower (B) as in FIG. 3, and herein, the second low-boiling-point material itself had a temperature of 45°C. After that, the second low-boiling-point material (8) was directly used as a feed for a separation tower (C), and with a total feed flow rate of 20 ton/hr, acetaldehyde was included in 9.1 wt%, the second absorbent (water) in 90.8 wt% and CO₂ in 0.11 wt% or less. Herein, the separation tower was operated at a pressure of 4.0 bar and a calorie (Q) of 2.38 Gcal/hr.

The second absorbent (water) and acetaldehyde (9) were separated in the separation tower (C), and with a final acetaldehyde flow rate of 1.86 ton/hr, acetaldehyde was included in 97.7 wt%, the second absorbent (water) in 2.2 wt% and CO₂ in 0.1 wt% or less, and after that, the separated second absorbent was supplied back to the absorption tower (B) of the step 2 after going through the heat exchanger (D) to which an additional heat source was supplied.

### <Example 2>

The first incompressible material (7) and the second low-boiling-point material (8) were separated in the absorption tower (B) as in FIG. 2, and herein, the second low-boiling-point material itself had a temperature of 61.2°C. After that, the second low-boiling-point material was introduced to a heat exchanger (D) and heated, and after the heating, the heated second low-boiling-point material (8-1) had a temperature of 127.8°C.

The heated second low-boiling-point material was used as a feed for a separation tower (C), and with a total feed flow rate of 8.8 ton/hr, acetaldehyde was included in 11.2 wt%, the second absorbent (water) in 87.4 wt%, acetic acid in 1.3 wt% and CO₂ in 0.1 wt% or less. Herein, the separation tower was operated at a pressure of 5.0 bar and a calorie (Q) of 0.57 Gcal/hr.

The second absorbent (water) and acetaldehyde (9) were separated in the separation tower (C), and with a final acetaldehyde flow rate of 1.02 ton/hr, acetaldehyde was included in 96.7 wt%, the second absorbent (water) in 3.2 wt% and CO₂ in 0.1wt% or less, and acetaldehyde was able to be commercialized as above.

The second absorbent (6) heated in the separation tower (C) was supplied back to the absorption tower (B) through a liquid flow, and herein, a calorie for cooling the heated second absorbent (6) was used as a heat source of the heat exchanger (D). Specifically, the second absorbent immediately after discharged from the separation tower (C) had a temperature of 151.9°C, and was cooled to 66.2°C as it is used as a heat source for heating the heat exchanger (D), and after that, was further cooled through cooling water and chilled water.

### <Comparative Example 2>

The first incompressible material (7) and the second low-boiling-point material (8) were separated in the absorption tower (B) as in FIG. 3, and herein, the second low-boiling-point material itself had a temperature of 61.2°C. After that, the second low-boiling-point material (8) was directly used as a feed for a separation tower (C), and with a total feed flow rate of 8.8 ton/hr, acetaldehyde was included in 11.2 wt%, the second absorbent (water) in 87.4 wt%, acetic acid in 1.3 wt% and CO₂ in 0.1 wt% or less. Herein, the separation tower was operated at a pressure of 5.0 bar and a calorie (Q) of 1.01 Gcal/hr.

The second absorbent (water) and acetaldehyde (9) were separated in the separation tower (C), and with a final acetaldehyde flow rate of 1.02 ton/hr, acetaldehyde was included in 96.7 wt%, the second absorbent (water) in 3.2 wt% and CO₂ in 0.1 wt% or less, and after that, the separated second absorbent was supplied back to the absorption tower (B) of the step 2 after going through the heat exchanger (D) to which an additional heat source was supplied.

### <Example 3>

The first incompressible material (7) and the second low-boiling-point material (8) were separated in the absorption tower (B) as in FIG. 2, and herein, the second low-boiling-point material itself had a temperature of 58°C. After that, the second low-boiling-point material was introduced to a heat exchanger (D) and heated, and after the heating, the heated second low-boiling-point material (8-1) had a temperature of 133.6°C.

The heated second low-boiling-point material was used as a feed for a separation tower (C), and with a total feed flow rate of 12.4 ton/hr, acetaldehyde was included in 6.8 wt%, the second absorbent (water) in 92.0 wt%, acrylic acid in 1.1 wt% and an inert gas such as CO₂, CO or N₂ in 0.1 wt% or less. Herein, the separation tower was operated at a pressure of 3.0 bar and a calorie (Q) of 0.70 Gcal/hr.

The second absorbent (water) and acetaldehyde (9) were separated in the separation tower (C), and with a final acetaldehyde flow rate of 0.886 ton/hr, acetaldehyde was included in 95.04 wt%, the second absorbent (water) in 4.88 wt% and CO₂ in 0.1 wt% or less, and acetaldehyde was able to be commercialized as above.

The second absorbent (6) heated in the separation tower (C) was supplied back to the absorption tower (B) through a liquid flow, and herein, a calorie for cooling the heated second absorbent (6) was used as a heat source of the heat exchanger (D). Specifically, the second absorbent immediately after discharged from the separation tower (C) had a temperature of 133.6°C, and was cooled to 63.0°C as it is used as a heat source for heating the heat exchanger (D), and after that, was further cooled through cooling water and chilled water.

### <Comparative Example 3>

The first incompressible material (7) and the second low-boiling-point material (8) were separated in the absorption tower (B) as in FIG. 3, and herein, the second low-boiling-point material itself had a temperature of 58°C. After that, the second low-boiling-point material (8) was directly used as a feed for a separation tower (C), and with a total feed flow rate of 12.4 ton/hr, acetaldehyde was included in 6.8 wt%, the second absorbent (water) in 92.0 wt%, acetic acid in 1.1 wt% and an inert gas such as CO₂, CO or N₂ in 0.1 wt% or less. Herein, the separation tower was operated at a pressure of 3.0 bar and a calorie (Q) of 1.20 Gcal/hr.

The second absorbent (water) and acetaldehyde (9) were separated in the separation tower (C), and with a final acetaldehyde flow rate of 0.886 ton/hr, acetaldehyde was included in 95.04 wt%, the second absorbent (water) in 4.88 wt% and CO₂ in 0.1 wt% or less, and after that, the separated second absorbent was supplied back to the absorption tower (B) of the step 2 after going through the heat exchanger (D) to which an additional heat source was supplied.

As identified in Examples 1 to 3 and Comparative Examples 1 to 3, the process for producing acrylic acid according to one embodiment of the present application includes the step 3 of heating the second low-boiling-point material including acetaldehyde (ACHO), and it was identified that, by increasing the temperature of the second low-boiling-point material discharged through the step 2 and introducing the result to the step 4, energy efficiency of the separation tower was able to be enhanced when separating in the step 4.

Specifically, it was identified that, with the calorie of the separation tower being 1.18 Gcal/hr in Example 1, 0.57 Gcal/hr in Example 2 and 0.70 Gcal/hr in Example 3, Examples 1 to 3 had lower calories compared to Comparative Examples 1 to 3, which prevented a load. Such results may be caused by heating the second low-boiling-point material in advance before introducing to the separation tower in Examples 1 to 3. In addition, in Examples 1 to 3, the second low-boiling-point material was heated through a heat exchanger, and it was identified that the heat exchanger used a cooling calorie of the heated second absorbent itself without requiring an additional heat source, and accordingly, the second low-boiling-point material was heated while cooling the second absorbent.

On the other hand, the second low-boiling-point material in an unheated state was introduced as it is in each of Comparative Examples 1 to 3, and a load on the separation tower itself was identified (2.38 Gcal/hr in Comparative Example 1, 1.01 Gcal/hr in Comparative Example 2, and 1.20 Gcal/hr in Comparative Example 3), and it was identified that an additional cooling calorie was required when supplying the additionally separated second absorbent back to the absorption tower (B) of the step 2 through a liquid circulation flow.

## Claims

1. A process for producing acrylic acid, the process comprising:
a step 1 of separating a first low-boiling-point material including acetaldehyde (ACHO) and a first high-boiling-point material including acrylic acid (AA) by adding a first absorbent to a reaction product of a bio-raw material and cooling the result;
a step 2 of separating a first incompressible material and a second low-boiling-point material including acetaldehyde (ACHO) by adding a second absorbent to the first low-boiling-point material including acetaldehyde (ACHO) and cooling the result;
a step 3 of heating the second low-boiling-point material including acetaldehyde (ACHO);
a step 4 of separating the heated second low-boiling-point material including acetaldehyde (ACHO) to acetaldehyde (ACHO) and the second absorbent; and
a step 5 of producing acrylic acid by purifying the first high-boiling-point material including acrylic acid (AA).

2. The process for producing acrylic acid of Claim 1, wherein the first absorbent includes a material having a boiling point difference of 20°C or higher compared to a normal boiling point (NBP) of the acrylic acid (AA) and a boiling point difference of 50°C or higher compared to a normal boiling point (NBP) of the acetaldehyde (ACHO).

3. The process for producing acrylic acid of Claim 1, wherein the second absorbent includes, as a material having a higher boiling point compared to a normal boiling point (NBP) of the acetaldehyde (ACHO), a material having a boiling point difference of 20°C or higher.

4. The process for producing acrylic acid of Claim 1, wherein the step 1 includes a step of separating through a cooling tower; and
the cooling tower has a cooling temperature of higher than or equal to 10°C and lower than or equal to 150°C and an inner pressure of greater than or equal to 0.5 bar and less than or equal to 5.0 bar.

5. The process for producing acrylic acid of Claim 1, wherein the step 2 includes a step of separating through an absorption tower; and
the absorption tower has a temperature of higher than or equal to 0°C and lower than or equal to 100°C and an inner pressure of greater than or equal to 0.1 bar and less than or equal to 10.0 bar.

6. The process for producing acrylic acid of Claim 1, wherein the step 3 includes a step of heating through a heat exchanger; and
as a heat source for the heating, a cooling calorie of the second absorbent of the step 4 is used as the heat source.

7. The process for producing acrylic acid of Claim 1, wherein the step 4 includes a step of separating through a separation tower; and
the separation tower has a temperature of higher than or equal to 10°C and lower than or equal to 200°C and an inner pressure of greater than or equal to 0.3 bar and less than or equal to 10.0 bar.

8. The process for producing acrylic acid of Claim 1, wherein the second absorbent has a temperature of higher than or equal to -5°C and lower than or equal to 20°C.

9. The process for producing acrylic acid of Claim 1, wherein the heated second low-boiling-point material including acetaldehyde (ACHO) has a temperature of higher than or equal to 60°C and lower than or equal to 180°C.

## Patentansprüche

1. Verfahren zur Herstellung von Acrylsäure, wobei das Verfahren umfasst:
einen Schritt 1 des Trennens eines ersten Materials mit niedrigem Siedepunkt, das Acetaldehyd (ACHO) enthält, und eines ersten Materials mit hohem Siedepunkt, das Acrylsäure (AA) enthält, durch Zugeben eines ersten Absorptionsmittels zu einem Reaktionsprodukt eines Biorohmaterials und Kühlen des Ergebnisses;
einen Schritt 2 des Trennens eines ersten inkompressiblen Materials und eines zweiten Materials mit niedrigem Siedepunkt, das Acetaldehyd (ACHO) enthält, durch Zugeben eines zweiten Absorptionsmittels zu dem ersten Material mit niedrigem Siedepunkt, das Acetaldehyd (ACHO) enthält, und Kühlen des Ergebnisses;
einen Schritt 3 des Erwärmens des zweiten Materials mit niedrigem Siedepunkt, das Acetaldehyd (ACHO) enthält;
einen Schritt 4 des Trennens des erwärmten zweiten Materials mit niedrigem Siedepunkt, das Acetaldehyd (ACHO) enthält, in Acetaldehyd (ACHO) und das zweite Absorptionsmittel; und
einen Schritt 5 der Herstellung von Acrylsäure durch Reinigen des ersten Materials mit hohem Siedepunkt, das Acrylsäure (AA) enthält.

2. Verfahren zur Herstellung von Acrylsäure nach Anspruch 1, wobei das erste Absorptionsmittel ein Material mit einer Siedepunktdifferenz von 20 °C oder höher im Vergleich zu einem normalen Siedepunkt (NBP) der Acrylsäure (AA) und einer Siedepunktdifferenz von 50 °C oder höher im Vergleich zu einem normalen Siedepunkt (NBP) des Acetaldehyds (ACHO) enthält.

3. Verfahren zur Herstellung von Acrylsäure nach Anspruch 1, wobei das zweite Absorptionsmittel als ein Material mit einem höheren Siedepunkt im Vergleich zu einem normalen Siedepunkt (NBP) des Acetaldehyds (ACHO) ein Material mit einer Siedepunktdifferenz von 20 °C oder höher enthält.

4. Verfahren zur Herstellung von Acrylsäure nach Anspruch 1, wobei der Schritt 1 einen Schritt des Trennens durch einen Kühlturm enthält; und
der Kühlturm eine Kühltemperatur von höher als oder gleich 10 °C und niedriger als oder gleich 150 °C und einen Innendruck von mehr als oder gleich 0,5 bar und weniger als oder gleich 5,0 bar aufweist.

5. Verfahren zur Herstellung von Acrylsäure nach Anspruch 1, wobei der Schritt 2 einen Schritt des Trennens durch einen Absorptionsturm enthält; und
der Absorptionsturm eine Temperatur von höher als oder gleich 0 °C und niedriger als oder gleich 100 °C und einen Innendruck von mehr als oder gleich 0,1 bar und weniger als oder gleich 10,0 bar aufweist.

6. Verfahren zur Herstellung von Acrylsäure nach Anspruch 1, wobei der Schritt 3 einen Schritt des Erwärmens durch einen Wärmetauscher enthält; und
als eine Wärmequelle für das Erwärmen eine Kühlkalorie des zweiten Absorptionsmittels des Schritts 4 als die Wärmequelle verwendet wird.

7. Verfahren zur Herstellung von Acrylsäure nach Anspruch 1, wobei der Schritt 4 einen Schritt des Trennens durch einen Trennturm enthält; und
der Trennturm eine Temperatur von höher als oder gleich 10 °C und niedriger als oder gleich 200 °C und einen Innendruck von mehr als oder gleich 0,3 bar und weniger als oder gleich 10,0 bar aufweist.

8. Verfahren zur Herstellung von Acrylsäure nach Anspruch 1, wobei das zweite Absorptionsmittel eine Temperatur von höher als oder gleich -5 °C und niedriger als oder gleich 20 °C aufweist.

9. Verfahren zur Herstellung von Acrylsäure nach Anspruch 1, wobei das erwärmte zweite Material mit niedrigem Siedepunkt, das Acetaldehyd (ACHO) enthält, eine Temperatur von höher als oder gleich 60 °C und niedriger als oder gleich 180 °C aufweist.

## Revendications

1. Procédé de production d'acide acrylique, le procédé comprenant :
une étape 1 de séparation d'un premier matériau à point d'ébullition bas incluant de l'acétaldéhyde (ACHO) et d'un premier matériau à point d'ébullition élevé incluant de l'acide acrylique (AA) en ajoutant un premier absorbant à un produit de réaction d'une matière première bio-dérivée et en refroidissant le résultat ;
une étape 2 de séparation d'un premier matériau incompressible et d'un deuxième matériau à point d'ébullition bas incluant de l'acétaldéhyde (ACHO) en ajoutant un deuxième absorbant au premier matériau à point d'ébullition bas incluant de l'acétaldéhyde (ACHO) et en refroidissant le résultat ;
une étape 3 de chauffage du deuxième matériau à point d'ébullition bas incluant de l'acétaldéhyde (ACHO) ;
une étape 4 de séparation du deuxième matériau à point d'ébullition bas chauffé incluant de l'acétaldéhyde (ACHO) en de l'acétaldéhyde (ACHO) et le deuxième absorbant ; et
une étape 5 de production de l'acide acrylique en purifiant le premier matériau à point d'ébullition élevé incluant de l'acide acrylique (AA).

2. Procédé de production d'acide acrylique selon la revendication 1, dans lequel le premier absorbant inclut un matériau ayant une différence de point d'ébullition égale ou supérieure à 20°C comparé à un point d'ébullition normal (NBP) de l'acide acrylique (AA) et une différence de point d'ébullition égale ou supérieure à 50°C comparé à un point d'ébullition normal (NBP) de l'acétaldéhyde (ACHO).

3. Procédé de production d'acide acrylique selon la revendication 1, dans lequel le deuxième absorbant inclut, en tant que matériau ayant un point d'ébullition plus élevé comparé à un point d'ébullition normal (NBP) de l'acétaldéhyde (ACHO), un matériau ayant une différence de point d'ébullition égale ou supérieure à 20°C.

4. Procédé de production d'acide acrylique selon la revendication 1, dans lequel l'étape 1 inclut une étape de séparation par une tour de refroidissement ; et
la tour de refroidissement a une température de refroidissement égale ou supérieure à 10°C et égale ou inférieure à 150°C et une pression interne égale ou supérieure à 0,5 bar et égale ou inférieure à 5,0 bar.

5. Procédé de production d'acide acrylique selon la revendication 1, dans lequel l'étape 2 inclut une étape de séparation par une tour d'absorption ; et
la tour d'absorption a une température égale ou supérieure à 0°C et égale ou inférieure à 100°C et une pression interne égale ou supérieure à 0,1 bar et égale ou inférieure à 10,0 bar.

6. Procédé de production d'acide acrylique selon la revendication 1, dans lequel l'étape 3 inclut une étape de chauffage par un échangeur de chaleur ; et
comme source de chaleur pour le chauffage, une calorie de refroidissement du deuxième absorbant de l'étape 4 est utilisée comme source de chaleur.

7. Procédé de production d'acide acrylique selon la revendication 1, dans lequel l'étape 4 inclut une étape de séparation par une tour de séparation ; et
la tour de séparation a une température égale ou supérieure à 10°C et égale ou inférieure à 200°C et une pression interne égale ou supérieure à 0,3 bar et égale ou inférieure à 10,0 bar.

8. Procédé de production d'acide acrylique selon la revendication 1, dans lequel le deuxième absorbant a une température égale ou supérieure à -5°C et égale ou inférieure à 20°C.

9. Procédé de production d'acide acrylique selon la revendication 1, dans lequel le deuxième matériau à point d'ébullition bas chauffé incluant de l'acétaldéhyde (ACHO) a une température égale ou supérieure à 60°C et égale ou inférieure à 180°C.
